# EUROPEAN PATENT SPECIFICATION

(11) **EP 0 334 015 B1**
(45) Date of publication and mention of the grant of the patent: **11.05.1994**
(21) Application number: 89102546.2
(22) Date of filing: 15.02.1989
(51) Int. Cl.: G01N 33/48, G01N 33/543, G01N 33/52, B01L 3/00

(54) **A device for delivering a fluid sample to a diagnostic device at a controlled rate**
Vorrichtung zur Abgabe einer flüssigen Probe auf ein diagnostisches Gerät in reguliertem Ausmass
Dispositif pour délivrer un échantillon fluide sur un support diagnostique à une vitesse contrôlée

(30) Priority: 23.03.1988 US 172108
(43) Date of publication of application: 27.09.1989
(73) Proprietor: Becton, Dickinson and Company, Franklin Lakes, New Jersey 07417-1880 (US)
(72) Inventor: Gary, Patricia A., Baltimore, MD (US); Maret, Melissa S., Damascus, MD (US)
(74) Representative: Selting, Günther, Dipl.-Ing.

(56) References cited:
- EP-A- 0 066 648
- EP-A- 0 217 403
- EP-A- 0 310 862

## Description

This invention pertains to the field of in vitro diagnostic testing devices. More particularly it relates to a device to deliver a fluid sample to a test device at a controlled rate.

Recent advances in immunology have opened a new class of diagnostic devices known as flow through devices. In general tests performed with these devices are much easier to perform than conventional immuno diagnostic assays. They require less precision in pipetting and fewer manipulations. They also eliminate the need for expensive instruments to read the results.

One such flow through device is described in US-A-4,632,901. The device shown there comprises a first member which is a membrane or filter to which is bound an antibody, or is capable of extracting cells or cell fragments from a fluid sample. The function of this first member is to trap the analyte on the membrane or filter. The device further comprises a second member which induces flow through the first member. In use a fluid sample and liquid reagents are applied to one surface of the first membrane. The reagents flow through the first member to the second member. Any analyte which is trapped on the first member by binding to a specific binding species for the analyte or by physical blockage of a cell or cell fragment associated with the analyte is then detected with a tracer system. The reagents for the tracer system are also applied to the first surface of the first member and flow through to the second member. The presence or absence of a detectable reaction on the first member is indicative of the presence or absence of analyte bound there.

Another flow through device is described in US-A-4,366,241. That device has an immunosorbing zone and a liquid receiving zone in liquid receiving relationship with the immunosorbing zone. The immunosorbing zone has one member of an immunological pair nondiffusively bound to it. In use a fluid sample and the reagents of a signal producing system are applied to the immunosorbing zone. The presence or absence of detectable reaction at the immunosorbing zone is indicative of the presence or absence of analyte in the sample.

US-A-4 366 241 describes several devices and assay protocols. The patent indicates that one or more layers may be interposed between the immunosorbing layer and the liquid absorbing zone. These layers function as barriers to inhibit back migration from the liquid absorbing layer to the immunosorbing layer; as fillers; for flow control; or the like.

One of the requirements of these flow through devices is sufficient exposure of the sample to the top layer to trap the analyte on the layer, typically with a specific binding reaction. Thereafter the reagents of the tracer must have sufficient exposure to the top layer for the appropriate reactions to occur. If the device wicks the fluids through the layers too quickly the reactions cannot occur. EP-A-0 310 862, which is prior art according to Article 54(3)EPC, addresses the problem of controlling flow across a porous support having a test area on its upper surface. The device described there has a porous layer with pores sufficiently large for unbound tracer and analyte to flow through to an absorptive layer. The porous layer and absorptive layer cooperate to control flow reagents through the porous layer into the absorptive layer. The device may also include a flow control layer positioned in between the porous layer and the absorptive layer.

A recently introduced kit to detect Group A streptococcus employs the invention described in EP-A-0 310 862. That kit includes all the reagents and supplies needed to detect the analyte in samples collected from a patient's throat. The sample is extracted in a DispensTube™ device. The extraction device is a test tube to which the sample and extraction reagents are added. After the sample has been extracted a dropper top is placed on the tube and the extracted sample can be dispensed to the test device. The dropper top includes a course filter to trap any large fragments present in the sample. Once the sample has been extracted the entire assay procedure can be conducted in as little as three to five minutes. The kit can detect Group A Strep. at quite low levels.

While the Directigen 1-2-3™ test kit (Becton Dickinson Microbiology Systems, Cokeysville, MD) described above works very well for Group A Strep, in assays for other analytes additional flow control is desirable.

Another problem that has arisen with the advent of very sensitive and specific assays is the problem of false positives arising out of the presence of a substance other than the analyte that binds to the specific binding species of the tracer and nonspecifically to the analyte immobilizing layer of a test system. Thus in assays using in the tracer system monoclonal antibodies derived from antisera obtained from mice, samples from people with antibodies to mice will give false positives. For this reason a need exists to eliminate the loss of effective specificity arising out of the presence of antibodies to the species used to make an antiserum used in the assay.

EP-A-0 217 403 describes a solid-phase analytical device comprising a planar layer of a material having a porous matrix of fibers and a plurality of substantially spherical, solid particles. The device comprises further a filtering means disposed in relationship to the sample-contacting surface of the planar layer. Dependent on the type of assay being performed, the filtering means is used as a "prefilter" to remove extraneous matter in a sample, or, for example, to separate and to hold blood cells from a whole blood sample while allowing plasma to pass through. It is a drawback of the known device that an additional flow control of the fluid sample delivered to the porous layer is not possible.

It is the object of the present invention to provide a device for delivering a fluid sample to a flow-through device at a controlled rate.

This problem is solved, according to the invention, with the features of claim 1.

The present invention is a device for delivering fluid samples to an in vitro diagnostic device at a controlled rate. It is comprised of a well for receiving a fluid sample, an opening in the bottom of the well and a flow controlling membrane covering the opening. The size of the opening and the mean pore size of the flow controlling membrane are selected so that a fluid in the well is delivered to a top surface of a diagnostic device at a controlled rate.

The device is constructed with the well and the opening in the bottom of the well being sized and shaped to mate with a test area of the test device. With this construction the device serves to deliver fluids across the entire surface of the test area at a controlled rate.

In a further aspect of the invention the device has means to selectively remove species that would interfere with the assay while allowing the analyte to flow out of the well. Preferably the means to remove interfering substances is a specific binding species that recognizes the interfering substance and does not bind the analyte. The specific binding species to remove the interfering substance is conveniently secured to the flow controlling membrane.

In yet another aspect of the invention the flow controlling membrane has reagents for the assay releasably coated on it. This allows dry storage of the reagents prior to use. Then as the sample and other fluids are passed through the flow controlling membrane, the reagents are eluted to the test device.

A most preferred construction of the device further comprises a handle secured to the well. The handle allows handling of the device without touching any fluids that have been placed in the well. Where the device is to be used and discarded before the tracer reagents are added to the diagnostic device the handle is particularly desirable.

The kit of the present invention comprises the flow controlling device described above and an in vitro diagnostic device matched to its fluid delivery capabilities. Thus systems can be developed where the fluid delivery device and a flow through diagnostic device are matched for a particular analyte and assay format.

In the method of the present invention a fluid sample is delivered to the well of the fluid delivery device and allowed to flow out of the opening across the flow controlling membrane to a test area of a diagnostic device. Thereafter reagents of a tracer system can be delivered to the test area through the fluid delivery device or independently of the fluid delivery device. Thus where the flow rate of a tracer conjugate across the test area is important to control, the fluid delivery device can be used to effect control. Similarly, in some systems multiple fluid delivery devices may be used to deliver species of dramatically different sizes at controlled rates.
Figure 1 is a side elevational view of the delivery device of the present invention;
Figure 2 is a cross section taken along section line 2 - 2 of Fig. 1;
Figure 3 is a top plan view of the preferred flow through diagnostic device for use in the kit of the present invention;
Figure 4 is an elevational view of the device shown in figure 3; and
Figure 5 is a sectional view of the flow through device shown in Fig. 3 taken along section line 5 - 5.

Referring now to the drawings, the delivery device of the present invention is comprised of a well 10 having at its top outwardly extending flanges 11 and depending sidewalls 12. The depending sidewalls 12 end at a bottom 13. An opening 14 in the bottom 13 allows a fluid in the well to flow out. The delivery device preferably has a handle 20 secured to the well 10. The handle 20 preferably has a section 21 with a cross section thinner than the remainder of the handle to make the handle readily flexible.1.

The well and handle are conveniently integrally formed by molding. The choice of material is not critical. Persons skilled in the art are familiar with suitable plastics. The well should be large enough to hold a fluid sample, e.g. 400 ul. The opening is sized to achieve the flow rate desired for the assay in question. It can conveniently have a diameter in the range of 0.05 to 0.22 in (0.13 cm to 0.56 cm). Preferably the opening has a diameter of 0.12 in (0.3 cm).

A flow controlling membrane 30 is secured to the bottom of the well. Flow controlling membranes are available from a variety of sources known to those skilled in the art. Presently preferred is a nylon 66 membrane having a mean pore size of 3.0 microns (Immunodyne™ I, Pall Corporation, East Hills, N.Y. 11548). The membrane can be secured to the well by any suitable method including with an adhesive, by heat sealing and by ultrasonic welding.

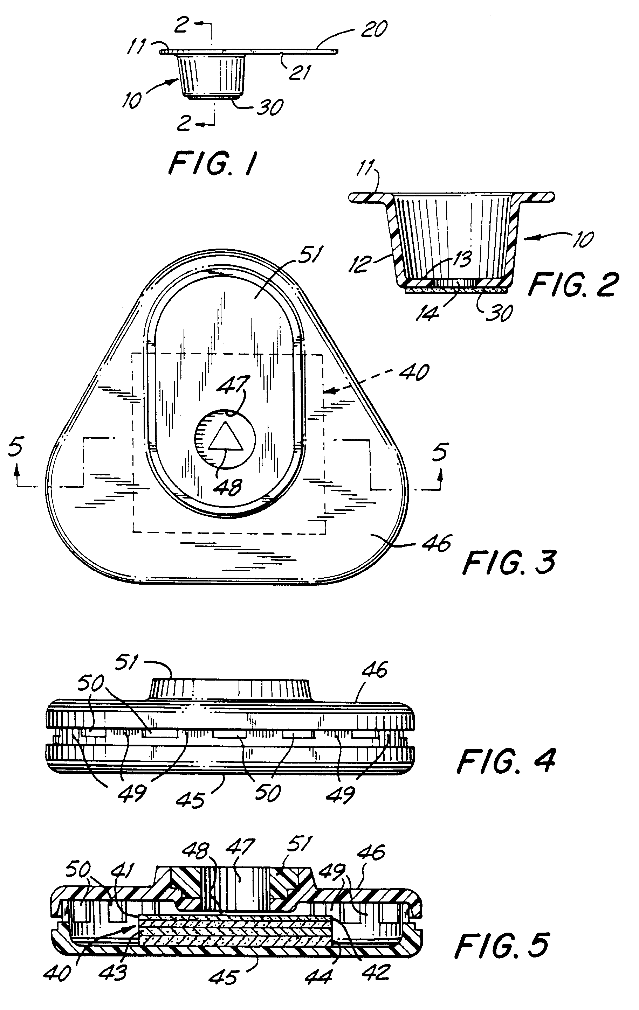

The preferred kit of the present invention uses the delivery device described above and the flow through Diagnostic device shown in Figs 3-5. The test device 40 is comprised of a porous support 41 having upper and lower surfaces and a test area 48 on its upper surface. Adjacent the lower surface of the porous support 41 is a flow control layer 42. Immediately underneath the flow control layer 42 is a porous spacer layer 43. Immediately underneath the porous spacer layer 43 is absorptive layer 44.

The layers 41, 42, 43, and 44 of test device may be attached to each other in any convenient way for example by sewing the layers to each other. The assembled device is conveniently placed within a container comprised of base 45 and cover 46. The cover 46 which overlies porous support 41 includes a raised portion having a suitable aperture 47 which overlies the test area 48. As shown in the figure the test area 48 is a triangle completely surrounded by a background portion of the porous support which is also within the area defined by aperture 47.

The cover 46 is supported over porous support 41 by teethlike projections 49 extending upward from the sides of the base 45. The projections 49 are of sufficient height to provide air spaces 50 which provide for ventilation of the sides of the test device 40.

The raised portion of the cover 46 surrounding the aperture 47 may include a colored area 51, the color of which contrasts from that of cover 46 and the color to be generated in the test area to provide for a better reading of the test results which are generally determined by color. In the preferred embodiment, the container comprised of base 45 and cover 46 having colored area 51 is made of plastic materials.

Preferably the delivery device is formed so that the well 10 fits in the aperture 47 in the container top. Most preferably the bottom of the well is sized and shaped to mate with the upper surface of the support 47. In use the fluid sample and any reagents for which flow control is desired are added to the well and allowed to flow through the membrane to the test area. When the delivery device is no longer needed it can be removed by the handle and discarded.

In another aspect of the invention the delivery device serves a further function of removing interfering substances from fluids to be used in the assay. This function may be accomplished by coating the interior walls of the well, the flow control membrane or both with a specific binding species that binds the interfering substance without binding the analyte and species active in the assay. Preferably, the flow control membrane is coated with a specific binding species. For example when the assay uses antibodies from mice, rabbits, or goats and very low levels of analyte area to be detected, antibodies in the patient's sera to the animal species used to make the reagents can cause false positive results. This problem also exists when monoclonal antibodies are used.

Further features and benefits of the invention will be apparent from the following nonlimiting examples.

### Comparative Example 1

### Preparation of Diagnostic Device

Schleicher & Schuell nitrocellulose membranes having a pore size of 5 microns are coated with 50 ul of a monoclonal antibody to cytoplasmic antigens (48-52 kD) of the fungus Candida Albicans described in U. S. Patent No. 4,670,382.0. The coating solution contains antibodies at 100 micrograms/ml in phosphate buffered saline (0.1 M, pH 6.0) containing 0.2 % NaN₃. After drying, the membrane is blocked with 0.5 % gelatin in phosphate buffered saline (0.1 M., pH 8.0). The membranes are then dried.

The test device is assembled by placing the nitrocellulose membrane on top of a layered composite. The two bottom layers are absorbent cellulose paper (1/4" thick); above these bottom layers is a porous spacer layer comprised of a nonwoven web of rayon (Schleicher & Schuell Cat. no 5-S). The three layers are stitched together and the nitrocellulose porous support layer is placed on top. The assembled composite is 1 square cm and is 0.5 cm thick. The antibody spot on the nitrocellulose membrane is in the shape of a triangle. The composite is placed in a container as shown in the figures.

### Delivery Device Preparation

A well and handle are molded from polystyrene resin (K-resin KR03, Phillips Petroleum, Bartlesville, OK). The well is cylindrical with an outside diameter of 0.395 in. (1.0 cm). Its capacity is 400 ul. The well is formed with a 0.12 in (0.3 cm) opening in its bottom. The well fits snuggly in the aperture of the flow through device.

A flow controlling membrane is heat sealed to the bottom of the well. It entirely covers the opening in the bottom of the well. The membrane is a nylon 66 membrane. Devices are made with membranes having a mean pore size of 3 microns and with membranes having a mean pore size of 1.2 microns (Immunodyne I™ Cat. No. B1A030HC5 and B1A012HC5 respectively, Pall Corporation, East Hills, NY).

### Test Suspension Preparation

Serum samples containing known concentration of cytoplasmic antigen (48-50kD) of the fungus Candida Albicans are prepared from pooled blood donor sera seeded with antigen at concentrations of 500 ng/ml, 200 ng/ml, 100 ng/ml, 50 ng/ml, and 25 ng/ml.

### Tracer Preparation

### A. Preparation of Liposome Particulate Label

1. To a 100 ml round-bottom rotoevaporator flask, add:
   a. 50 mg cholesterol (Sigma #CH-S),
   b. 94 mg distearoyl phosphatidyl choline, (Avanti Polar Lipids #850365),
   c. 10 mg distearoyl phosphatididyl glycerol (Avanti Polar Lipids),
   d. 3.75 mg crosslinking agent (distearoyl phosphatidyl ethanol-amine-p- maleimidophenyl) capryl (Becton Dickinson Immunodiagnostic, Orangeburg, N.Y.); and
   e. 50 ml chloroform (Fisher).
2. Swirl to mix.
3. Place on rotoevaporator with the following settings:
   Water bath temperature = 44°C
   Rotation speed = 4
4. Slowly increase vacuum until foaming ceases (approximately 30-40 min).
5. Reduce pressure and allow liposomes to anneal at 44°C for 30 min.
6. Lypholize overnight.
7. On a rotoevaporator add 50 ml distilled water and stir at 60°C without vacuum until lipid film is dissolved.
8. Freeze in dry ice and methanol.
9. Lypholize to a dry powdered liposome.
10. Separately prepare a colored solution of Sulfurhodamine B (0.1M in sodium acetate saline buffer, 0.1M, pH 4.5).
11. Add 50 ml of the colored solution to the liposome powder and warm to 60°C for 15 minutes.
12. Extrude the warm liposome preparation through a 1.0 micron, a 0.4 micron and then a 0.2 micron Biorad Unipore polycarbonate membrane (Biorad).
13. Separate free colored material from the liposome suspension on a Sepharose CL6B chromotography column (Pharmacia) equilibrated in 50 mM sodium acetate buffer pH, 4.5 with 1 mM EDTA and 50 mM NaCl.
14. Store liposomes in the buffer specified in step 13.

### B. Coupling of Liposome Particulate Label to Specific Binding Species

1. goat antibody to Rabbit (6 mg, Jackson Immuno Research, Westgrove, PA) in phosphate buffered saline (100 mM, pH 7.5) is mixed with SPDP (Sigma) at a molar ratio of 6.6 ug SPDP: 1 mg antibody; the mixture is flushed with nitrogen and sealed. It is allowed to react for thirty minutes at room temperature with stirring.
3. Add 1/10th volume of 1 M sodium acetate pH 4.5 stir for 30 seconds.
4. Add 1/100th volume of 1 M dithiothreitol in water.
5. Remove dithiothreitol by passing the reaction volume over a Sephadex G-25 medium column equilibrated with Tris buffer (50 mM Tris, 50 mM sodium acetate 50 mM NaCl, 1 mM EDTA, pH 8.0). Flush with nitrogen and seal.
6. Monitor the O.D. 280 and pool protein containing fractions.
7. Mix this solution with the 10 ml of freshly prepared liposomes. The amount of liposomes is determined by ratio of 20 uM phosphorous to 1.25 mg of recovered protein. Phosphorous determination may vary from prep to prep of liposomes.
8. Flush with N₂ and seal.
9. React 2 hours overnight at room temperature.
10. Separate coupled product on a Sepharose Fast Flow™ chromatography column (Pharmacia) equilibrated with standard borate buffer (pH 8).
11. Collect and pool void volume fraction.
12. Store at 4°C.

### Assay Procedure

Assays are run with and without the delivery device. Where no delivery device is used the test suspension and all reagents are added directly to the test area of the diagnostic device. When the delivery device is used it is inserted into the aperture of the diagnostic device and a test suspension (200 ul) is placed in the the well; it flows through to the test area and from there through to the absorptive layer. An aliquot of rabbit antibody against cytoplasmic antigen (48-52 kD) of the fungus Candida Albicans (150 ul, 300 ug/ml) is then placed in the well of the delivery device and allowed to flow through to the test area. Thereafter the delivery device is removed from the diagnostic device and discarded. The tracer (150 ul) is added.

The test area is then washed with the wash buffer (0.1M quanidine HCl) and the results are then read by visually observing the presence of a distinctive pink color (triangle) on the test area.

The total time to perform each assay is three to five minutes. In Table I the symbol "++" signifies the presence of a distinctive pink color on the test area, the symbol "+" signifies a faint pink color and the symbol "-" means that no color is observed. The use of the delivery device substantially improves the sensitivity of the assay, from 200 ng/ml to 25 ng/ml.

**Table I**

| Assay Format | Antigen Conc. ng/ml | | | | |
|---|---|---|---|---|---|
| | 500 | 200 | 100 | 50 | 25 |
| no delivery device | ++ | + | - | | |
| 3 micron device | ++ | ++ | ++ | ++ | + |
| 1.2 micron device | ++ | ++ | ++ | ++ | + |

### Example 2

### Preparation of Diagnostic Device

Nitrocellulose membranes having a mean pore size of 5 microns (MSI, West Borough, MA) are coated with 50 ul of a monoclonal antibody to cytoplasmic antigen (48-52 kD) of the fungus Candida Albicans described in U.S. Patent No. 4,670,382.0. The coating solution contains antibodies at 75 micrograms/ml in phosphate buffered saline (0.1 M, pH 6.0) containing 0.2 % NaN₃. After drying, the membrane is blocked with 0.5 % gelatin in phosphate buffered saline (0.1 M., pH 8.0). The membranes are then dried.

The test device is assembled by placing nitrocellulose membranes on top of a layered composite. The two bottom layers are absorbent cellulose paper (1/4" thick); above these bottom layers is a porous spacer layer comprised of a nonwoven web of rayon (Schleicher & Schuell Cat. no 5-S). Above the rayon layer is a polycarbonate unidirectional flow controlling membrane having a mean pore size of 1.0 um (Nuclepore, Pleasantville, CA). The four layers are stitched together and the nitrocellulose porous support layer is placed on top. The assembled composite is 1 square cm and is 0.5 cm thick. The antibody spot on the nitrocellulose membrane is in the shape of a triangle. The composite is placed in a container as shown in the figures.

### Assay Procedure

Assays were performed using delivery devices and tracer as described in Comparative Example 1. With the delivery device inserted in the aperture of the diagnostic device a 200 ul sample of serum seeded with 10 ng/ml of cytoplasmic antigen (48-52 kD) of the fungus Candida Albicans is added and allowed to flow through to the diagnostic device. An aliquot of rabbit antibody against the antigen (150 ul, 75 ug/ml) is then placed in the well of the delivery device and allowed to flow through to the test area. Thereafter the delivery device is removed and discarded. The tracer (150 ul) is added to the test area. After the tracer has flowed through the test device, the test area is washed with wash buffer (0.1 M quanidine HCl) and the results are read by visually observing the presence of a distinctive pink triangle on the test area. When the experiment was repeated using serum not seeded with antigen a negative result was observed.

### Example 3

### Preparation of Delivery Device

A well and handle are molded from polystyrene resin (K-resin KR03, Phillips Petroleum, Bartlesville, OK). The well is cylindrical with an outside diameter of 0.395 in. (1.0 cm). Its capacity is 400 ul. The well is formed with a 0.12 in (0.3 cm) opening in its bottom. The well fits snuggly in the aperture of the flow through device.

A flow controlling membrane is heat sealed to the bottom of the well. It entirely covers the opening int he bottom of the well. The membranes used were nylon 66 (Immunodyne I™, Pall Corporation , East Hills, NY) or nitrocellulose (MSI, West Borough, Mass.). The flow controlling membranes in the delivery devices were treated with blocking solutions as follows:
- Device 1 --: 3.0 micron mean pore size nylon membrane blocked with 1 % nonfat dried milk.
- Device 2 --: 3.0 micron mean pore size nylon membrane blocked with 10 % mouse serum.
- Device 3 --: 5.0 micron mean pore size nitrocellulose membrane blocked with 10 % mouse serum.

### Assay Procedure

Assays were performed using diagnostic devices, tracer and procedure as described in Example 2. The sample was a normal human serum which gives a false positive result. When the assay was performed with the delivery device having its flow controlling membrane blocked with non fat dry milk a positive result was observed. When it was performed with the two delivery devices blocked with mouse serum, the results were negative.

## Claims

1. A device for delivering a fluid sample to a flow-through diagnostic test assembly having a porous support layer (41) with an upper surface wherein a specific shaped test area (48) is on the upper surface of said support (41) while the upper surface of said support (41) without the test area surrounds the test area, at a controlled rate, comprising:
a well (10) for receiving the fluid sample, comprising side walls (12) that end in a bottom (13), an opening (14) in the bottom of the well sized and shaped the same as the specific shaped test area (48) in the flow-through diagnostic test assembly, and
a flow controlling membrane (30) attached to the well (10) and covering the opening (14) to deliver the fluid sample to said specific shaped test area (48) on the upper surface of said porous support (41) of the flow-through diagnostic test assembly at a controlled rate.

2. The device of claim 1 further comprising means in the device to selectively remove interfering substances from a fluid sample without trapping an analyte in the fluid sample.

3. The device of claim 2 wherein the means to selectively remove interfering substances is a specific binding species for the interfering substance.

4. The device of claim 3 wherein the specific binding species is secured to the flow controlling membrane (30).

5. The device of claim 1 wherein the flow controlling membrane (30) has assay reagents releasably coated on it.

6. The device of claim 1 further comprising a handle (20) secured to the well (10) to facilitate handling of the device without touching a fluid sample in the well.

7. A diagnostic kit comprised of the device of claim 1 and a flow-through diagnostic test assembly.

8. A diagnostic kit of claim 7 wherein said flow-through diagnostic test assembly comprises a casing having an aperture and a porous support (41) comprising an upper surface and a specific shaped test area (48) on the upper surface of said support (41) while the upper surface of the support without the test area surrounds the test area.

9. An assay for an analyte in a fluid sample comprising:
mating the well (10) of the device of claim 1 with the flow-through diagnostic test assembly,
delivering the fluid sample to the device,
directing the fluid sample out of the device at a controlled rate,
contacting the specific shaped test area (48) on the upper surface of the support layer (41) of said assembly with the sample, while the upper surface of the support layer, without the test area is free of said fluid sample, wherein said specific shaped test area (48) is a specific binding species for the analyte without interference from said support, and
detecting the presence of the analyte in the fluid sample that has been delivered on the specific shaped test area (48) using a tracer.

## Patentansprüche

1. Vorrichtung zum Ausgeben einer Fluidprobe an eine diagnostische Durchfluß-Testanordnung mit geregelter Fließrate, wobei die Testanordnung eine poröse Trägerschicht (41) mit einer Oberseite aufweist, bei der eine spezifisch geformte Testfläche (48) auf der Oberseite des Trägers (41) vorgesehen ist, während die nicht mit der Testfläche (48) versehene Oberseite des Trägers (41) die Testfläche umgibt, mit:
einem Napf (10) zum Aufnehmen der Fluidprobe, mit Seitenwänden (12), die in einem Boden (13) enden, einer Öffnung (14) im Boden des Napfs, die dieselbe Größe und Form aufweist wie die spezifisch geformte Testfläche (48) in der diagnostischen Durchfluß-Testanordnung; und
einer Flußregelmembran (30), die an dem Napf (10) angebracht ist und die Öffnung (14) bedeckt, um die Fluidprobe auf die spezifisch geformte Testfläche (48) an der Oberseite des porösen Trägers (41) der diagnostischen Durchfluß-Testanordnung mit geregelter Fließrate auszugeben.

2. Vorrichtung nach Anspruch 1, ferner mit einer in der Vorrichtung vorgesehenen Einrichtung zum selektiven Entfernen störender Substanzen aus einer Fluidprobe, ohne einen Analyten in der Fluidprobe einzuschließen.

3. Vorrichtung nach Anspruch 2, bei der die Einrichtung zum selektiven Entfernen störender Substanzen eine spezifische Bindemittelart für die störende Substanz ist.

4. Vorrichtung nach Anspruch 3, bei der die spezifische Bindemittelart an der Flußregelmembran (30) angebracht ist.

5. Vorrichtung nach Anspruch 1, bei der die Flußregelmembran (30) mit Assay-Reagenzien lösbar beschichtet ist.

6. Vorrichtung nach Anspruch 1, ferner mit einem an dem Napf (10) vorgesehenen Griff (20), um das Handhaben der Vorrichtung ohne Berühren einer Fluidprobe in dem Napf zu erleichtern.

7. Diagnose-Kit bestehend aus der Vorrichtung nach Anspruch 1 und einer diagnostischen Durchfluß-Testanordnung.

8. Diagnose-Kit nach Anspruch 7, bei dem diagnostische Durchfluß-Testanordnung ein Gehäuse mit einer Öffnung und einen porösen Träger (41) mit einer Oberseite aufweist, wobei auf der Oberseite des Trägers (41) eine spezifisch geformte Testfläche (48) ausgebildet ist, während die nicht mit einer Testfläche versehene Oberseite des Trägers die Testfläche umgibt.

9. Assay für einen Analyten in einer Fluidprobe mit den folgenden Schritten:
Zusammensetzen des Napfs (10) der Vorrichtung nach Anspruch 1 mit der diagnostischen Durchfluß-Testanordnung,
Ausgeben der Fluidprobe an die Vorrichtung,
Leiten der Fluidprobe aus der Vorrichtung mit geregelter Fließrate,
die spezifisch geformte Testfläche (48) auf der Oberseite der Trägerschicht (41) der Anordnung in Kontakt mit der Probe bringen, wobei die die Testfläche nicht aufweisende Oberseite der Trägerschicht von der Fluidprobe frei ist, wobei die spezifisch geformte Testfläche (48) eine spezifische Bindemittelart für den Analyten ohne Störung durch den Träger ist, und
Erkennen des Vorhandenseins des Analyten in der auf die spezifisch geformte Testfläche (48) aufgebrachte Fluidprobe unter Verwendung eines Tracers.

## Revendications

1. Dispositif permettant de délivrer un échantillon de fluide à un ensemble d'essai diagnostique à flux traversant ayant une couche de support poreuse (41) avec une surface supérieure sur laquelle se trouve une zone d'essai de forme spécifique (48) tandis que la surface supérieure dudit support (41) sans la zone d'essai entoure celle-ci, à vitesse régulée, le dispositif comprenant :
un puits (10) pour recevoir l'échantillon de fluide, comprenant des parois latérales (12) qui se terminent par un fond (13), une ouverture (14) dans le fond, de même calibre et de même forme que celle de la zone d'essai de forme spécifique (48) de l'ensemble d'essai diagnostique à flux traversant, et
une membrane de régulation de flux (30) fixée au puits (10) et recouvrant l'ouverture (14) pour délivrer l'échantillon de fluide à ladite zone d'essai de forme spécifique (48) sur la surface supérieure dudit support poreux (41) de l'ensemble d'essai diagnostique à flux traversant à vitesse réglée.

2. Dispositif selon la revendication 1 comprenant par ailleurs un moyen dans le dispositif pour éliminer sélectivement les substances interférentes provenant d'un échantillon de fluide sans emprisonner une analyte dans l'échantillon de fluide.

3. Dispositif selon la revendication 2, dans lequel le moyen d'élimination sélective des substances interférentes est formé par une espèce donnant une liaison spécifique pour la substance interférente.

4. Dispositif selon la revendication 3, dans lequel l'espèce donnant la liaison spécifique est fixé à la membrane de régulation du flux (30).

5. Dispositif selon la revendication 1, dans lequel la membrane de régulation du flux (30) comporte des réactifs d'essai qui recouvrent la membrane de manière libérable.

6. Dispositif selon la revendication 1, comprenant par ailleurs une manette (20) fixée au puits (10) pour faciliter la manipulation du dispositif sans toucher l'échantillon de fluide dans le puits.

7. Trousse diagnostique constituée du dispositif de la revendication 1 et d'un ensemble d'essai diagnostique à flux traversant.

8. Trousse diagnostique selon la revendication 7, dans laquelle l'ensemble d'essai diagnostique à flux traversant comprend un boîtier ayant une ouverture et un support poreux (41) comprenant une surface supérieure et une zone d'essai de forme spécifique (48) à la surface supérieure dudit support (41) tandis que la surface supérieure dudit support sans la zone d'essai entoure celle-ci.

9. Essai de titrage d'analyte dans un échantillon de fluide, comprenant les étapes suivantes :
on accouple le puits (10) du dispositif de la revendication 1 avec l'ensemble d'essai diagnostique à flux traversant,
on délivre l'échantillon de fluide au dispositif,
on dirige l'échantillon de fluide hors du dispositif à vitesse régulée,
on met en contact la zone d'essai de forme spécifique (48) sur la surface supérieure de la couche de support (41) dudit ensemble avec l'échantillon, tandis que la surface supérieure de la couche de support, sans la zone d'essai, est exempte dudit échantillon de fluide, ladite zone d'essai de forme spécifique (48) étant constituée d'une espèce donnant une liaison spécifique pour l'analyte sans interférence à partir dudit support, et
on détecte la présence de l'analyte dans l'échantillon de fluide qui a été délivré sur la zone d'essai de forme spécifique (48) en utilisant un traceur.
